# EUROPÄISCHE PATENTSCHRIFT

(11) **EP 1 904 511 B1**
(45) Veröffentlichungstag und Bekanntmachung des Hinweises auf die Patenterteilung: **08.07.2009**
(21) Anmeldenummer: 06760795.2
(22) Anmeldetag: 20.07.2006
(51) Int. Cl.: C07H 19/073, A61K 31/7068

(54) **HOMOGEMCITABINE**
HOMOGEMCITABINES
HOMOGEMCITABINES

(30) Priorität: 20.07.2005 AT 12242005
(43) Veröffentlichungstag der Anmeldung: 02.04.2008
(73) Patentinhaber: Pharmacon-Forschung und Beratung GmbH, 4780 Brunnenthal (AT)
(72) Erfinder: NOE, Christian, R., A-1180 Wien (AT); JASIC, Muhamed, A-1180 Wien (AT); KOLLMANN, Hermann, A-4020 Linz (AT); SAADAT, Karmin, A-1190 Wien (AT)
(74) Vertreter: Sonn & Partner Patentanwälte
(86) Internationale Anmeldenummer: PCT/AT2006/000308
(87) Internationale Veröffentlichungsnummer: WO 2007/009147

(56) Entgegenhaltungen:
- US-A- 5 744 597
- HERTEL L W ET AL: "EVALUATION OF THE ANTITUMOR ACTIVITY OF GEMCITABINE (2',2'-DIFLUORO-2'-DEOXYCYTIDINE)" CANCER RESEARCH, AMERICAN ASSOCIATION FOR CANCER RESEARCH, BALTIMORE, MD, US, Bd. 50, 15. Juli 1990 (1990-07-15), Seiten 4417-4422, XP000919489 ISSN: 0008-5472
- CHOU T S ET AL: "STEREOSPECIFIC SYNTHESIS OF 2-DEOXY-2,2-DIFLUORORIBONOLACTONE AND ITS USE IN THE PREPARATION OF 2'-DEOXY-2'.2'-DIFLUORO-BETA-D-RIBOFURANOS YL PYRIMIDINE NUCLEOSIDES: THE KEY ROLE OF SELECTIVE CRYSTALLIZATION" SYNTHESIS, GEORG THIEME VERLAG, STUTTGART, DE, Nr. 6, 1. Juni 1992 (1992-06-01), Seiten 565-570, XP000572747 ISSN: 0039-7881

## Beschreibung

Die vorliegende Erfindung betrifft Homogemcitabine, Verfahren zu ihrer Herstellung, ihre Verwendung zur Herstellung des Wirkstoffes Gemcitabin und ihre Verwendung zur Herstellung von Arzneimitteln zur Behandlung proliferativer Erkrankungen. Weiters betrifft die vorliegende Erfindung neue Zwischenverbindungen, wie sie im erfindungsgemäßen Verfahren Verwendung finden.

Der Wirkstoff Gemcitabin der Formel 1 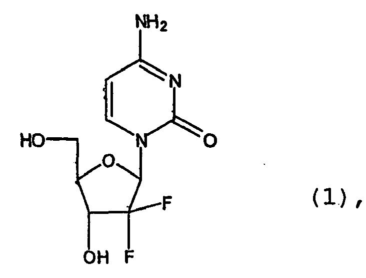 ist mittlerweile als Antimetabolit zur Behandlung von Krebserkrankungen erfolgreich etabliert (Hertel et al., Cancer Research, 50, 1990, 4417-4422). Da die Herstellung dieses im Zuckerteil zweifach fluorierten Nucleosids mit einem beträchtlichen synthetischen Aufwand verbunden ist, sind mittlerweile etliche Arbeiten bekannt geworden, welche sich vor allem mit der Verbesserung der ursprünglich publizierten Synthese, welche von geschütztem Glycerinaldehyd ausgeht, befassen. Der zentrale Schritt bei dieser Synthese ist die Nukleosidierung eines fluorierten Pentosederivates mit der aktivierten Nucleobase Cytosin (Chou et al., Synthesis, Nr. 6, 1992, 565-570; US-A-5 744 597).

Ein erster Gegenstand der vorliegenden Erfindung sind Homologe des Gemcitabins der allgemeinen Formel 2, 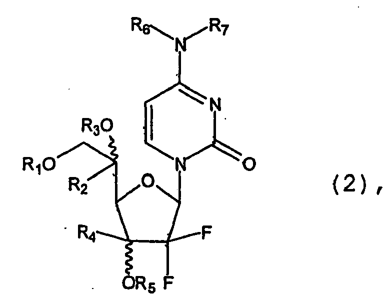 worin R₁, R₃ und R₅ Wasserstoff oder eine geeignete Hydroxyschutzgruppe, wie sie im Stand der Technik etwa aus Protective Groups in Organic Synthesis (Greene, Wuts), 3. Auflage, John Wiley & Sons, Inc., Seiten 17 bis 245, bekannt sind, insbesondere eine Benzoylgruppe bedeuten, R₂ und R₄ jeweils Wasserstoff oder Alkyl mit 1 bis 6 C-Atomen bedeuten, und R₆ und R₇ jeweils Wasserstoff oder eine geeignete Aminoschutzgruppe, wie sie im Stand der Technik etwa aus Protective Groups in Organic Synthesis (Greene, Wuts), 3. Auflage, John Wiley & Sons, Inc., Seiten 494 bis 653, bekannt sind, insbesondere Acetyl, Alkylsilanyl oder Arylalkylsilanyl mit 1 bis 6 C-Atomen im Alkylteil bedeuten und die gewellte Linie jeweils beide mögliche Konfigurationen von -OR₃ bzw. -OR₅ in Bezug auf den Grundkörper bedeutet.

Verbindungen der allgemeinen Formel 2, insbesondere worin R₁ bis R₇ jeweils Wasserstoff bedeuten, sind interessante Kandidaten als Therapeutika proliferativer Erkrankungen, wobei die Verwendung der Homogemcitabine der allgemeinen Formel 2, zur Herstellung von Arzneimitteln zur Behandlung proliferativer Erkrankungen einen weiteren Gegenstand der vorliegenden Erfindung darstellt. Insbesondere wird angenommen, dass Verbindungen der allgemeinen Formel 2 zur Herstellung von Arzneimitteln zur Behandlung von NSCLC (non-small cell lung cancer, Nicht-kleinzelliges Lungenkarzinom), Mammakarzinom, Ovarialkarzinom, Pankreaskarzinom und Harnblasenkarzinom geeignet sind, und zwar alleine oder in Kombination mit anderen Wirkstoffen/Medikamenten. Die etablierten Therapieschemata sehen meist Kombinationen verschiedener Cytostatika vor, z.B. wird bei Blasenkarzinom mit Cisplatin kombiniert, bei Ovarialkarzinom mit Carboplatin.

Ein weiterer Gegenstand der vorliegenden Erfindung ist ein Verfahren zur Herstellung von derartigen Homologen des Gemcitabins der allgemeinen Formel 2. Diese Verbindungen lassen sich beispielsweise durch Nukleosidierung geeigneter fluorierter und gegebenenfalls geschützter Hexosen der allgemeinen Formel 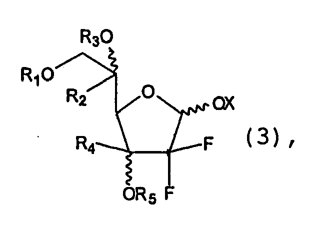 in welcher R₁ bis R₅ die oben angegebene Bedeutung haben, X Wasserstoff oder eine an sich bekannte Aktivierungsgruppe, vorzugsweise einen Alkylsulfonylrest mit 1 bis 6 C-Atomen im Alkylteil darstellt und die gewellte Linie jeweils beide mögliche Konfigurationen von OX, -OR₃ bzw. -OR₅ in Bezug auf den Grundkörper bedeutet,
mit einem mit Schutzgruppen versehenen Cytosin der allgemeinen Formel 4 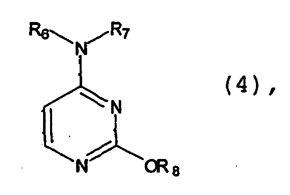 in welcher R₆ und R₇ die oben angegebenen Bedeutungen haben, wobei vorzugsweise mindestens eine der beiden Gruppen Trialkylsilanyl oder Triarylalkylsilanyl mit jeweils 1 bis 6 C-Atomen im Alkylteil bedeutet und R₈ eine geeignete Abgangsgruppe, vorzugsweise gleich mit R₆ und R₇, ist, herstellen, wobei anschließend gegebenenfalls allfällige noch vorhandene Schutzgruppen abgespalten werden um Verbindungen der allgemeinen Formel (2) zu erhalten, worin R₁ bis R₇ jeweils Wasserstoff bedeuten.

Die oben erwähnten fluorierten Hexosen der allgemeinen Formel 3 sind neue Verbindungen und stellen auch einen weiteren Gegenstand der vorliegenden Erfindung dar. Sie lassen sich ihrerseits durch Addition einer difluorierten Komponente an einen enantiomerenreinen C4-Baustein, welcher mit einem seiner Chiralitätszentren die D-Konfiguration gemäß der Kohlenhydratnomenklatur vorgibt, herstellen, vorzugsweise durch Addition eines difluorierten Essigsäurederivates der allgemeinen Formel 5, 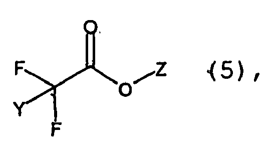 in welcher Y eine geeignete Abgangsgruppe, wie Brom, Chlor oder Jod, oder Wasserstoff, und Z Alkyl mit 1 bis 6 C-Atomen bedeuten, an ein geschütztes Derivat einer L-Threose oder D-Erythrose der allgemeinen Formel 6 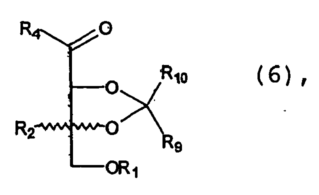 worin R₁, R₂ und R₄ wie oben definiert sind und R₉ und R₁₀ jeweils Wasserstoff, eine Alkylgruppe mit 1 bis 3 C-Atomen oder Phenyl bedeuten.

Das Additionsprodukt wird anschließend zum Lacton der allgemeinen Formel 3, 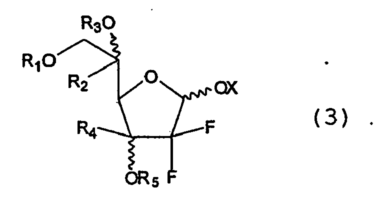 in welcher R₁, R₂, R₄ und R₅ die oben angegebene Bedeutung haben, R₃ Wasserstoff darstellt und die gewellte Linie zusammen mit OX eine Ketogruppe bedeutet, zyklisiert.

Zur Herstellung von Homogemcitabinen werden dann vorhandene freie Hydroxylgruppen der Verbindungen der allgemeinen Formel 3 durch Verwendung einer geeigneten Hydroxyschutzgruppe, wie sie im Stand der Technik etwa aus Protective Groups in Organic Synthesis (Greene, Wuts), 3. Auflage, John Wiley & Sons, Inc., Seiten 17 bis 245, bekannt sind, vorzugsweise mit einer gegebenenfalls substituierten Benzoylgruppe, erneut geschützt. Das geschützte Lacton wird hydriert, beispielsweise mit Hilfe eines komplexen Hydrids, und so in das Lactol der allgemeinen Formel 3, in welcher R₁, R₃ und R₅ jeweils geeignete Hydroxyschutzgruppen, vorzugsweise gegebenenfalls substituierte Benzoylgruppen, darstellen und X Wasserstoff bedeutet, übergeführt, welches Lactol für die anschließende Nukleosidierungsreaktion aktiviert wird, wobei diese Aktivierung vorzugsweise durch Einführung eines Alkylsulfonylrestes mit 1 bis 6 C-Atomen im Alkylteil erfolgt. Das aktivierte Lactol wird dann mit einem mit Schutzgruppen versehenen Cytosin der allgemeinen Formel 4 umgesetzt, anschließend werden gegebenenfalls allfällige noch vorhandene Schutzgruppen abgespalten um Verbindungen der allgemeinen Formel (2) zu erhalten, worin R₁ bis R₇ jeweils Wasserstoff bedeuten.

Insbesondere sind die neuen Verbindungen der allgemeinen Formel 3 auch gut als Ausgangsprodukte zur Herstellung von Gemcitabin geeignet. Zu diesem Zweck wird die Verbindung der allgemeinen Formel 3, wie bereits beschrieben mit einem mit Schutzgruppen versehenen Cytosin der allgemeinen Formel 4 zu einer Verbindung der allgemeinen Formel 2 nukleosidiert, gegebenenfalls die Schutzgruppen an den Hydroxylgruppen des Zuckerteils und am Cytosin entfernt und eine Verbindung der Formel 2 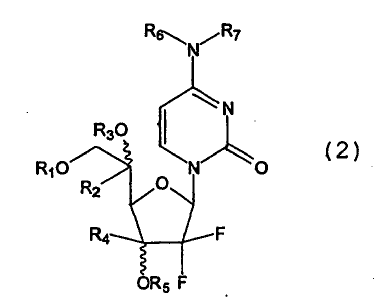 erhalten, worin R₁ bis R₇ jeweils Wasserstoff bedeuten und die gewellte Linie jeweils beide mögliche Konfigurationen von -OR₃ bzw. -OR₅ in Bezug auf den Grundkörper bedeutet, diese wird dann einer Glykolspaltung zum Aldehyd der Formel 7 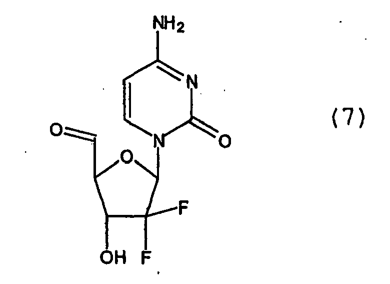 unterzogen, wobei in der Glykolspaltung sowohl das reine ß-Anomere von Verbindungen der allgemeinen Formel 2 worin R₁ bis R₇ jeweils Wasserstoff bedeuten als auch das bei der Nukleosidierung entstehende Gemisch des α- und ß-Anomeren eingesetzt werden kann. Die Glykolspaltung wird dabei mit den üblichen Reagentien, vorzugsweise mit einem Perjodat, durchgeführt, worauf die Aldehydgruppe des Aldehyds 7 mit einem komplexen Hydrid, vorzugsweise mit Natriumborhydrid, zur Hydroxygruppe reduziert wird. Die Reduktion wird am besten in einer Eintopfreaktion im Anschluss an die Glykolspaltung durchgeführt, wodurch Gemcitabin der Formel 1 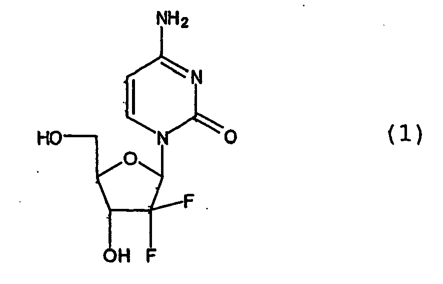 unmittelbar erhalten und durch Umkristallisation in ein Produkt pharmazeutischer Qualität umgewandelt werden kann.

Einen weiteren Gegenstand der vorliegenden Erfindung bildet ein Verfahren zur Herstellung von Verbindungen der allgemeinen Formel 2 durch Fluorierung von Ketohexosenukleosiden der allgemeinen Formel 12 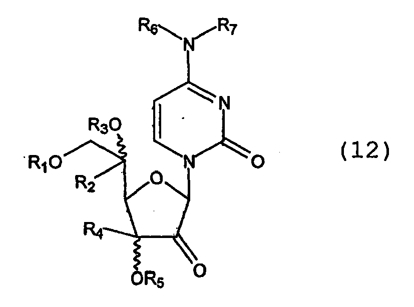 mit einem geeigneten Fluorierungsmittel, vorzugsweise mit DAST (Diethylaminoschwefeltrichlorid) in Kombination mit HF.

Auch die Ketohexosenukleoside der allgemeinen Formel 12 sind neue Verbindungen und stellen noch einen weiteren Gegenstand der vorliegenden Erfindung dar. Herstellen lassen sie sich indem eine Verbindung der allgemeinen Formel 8 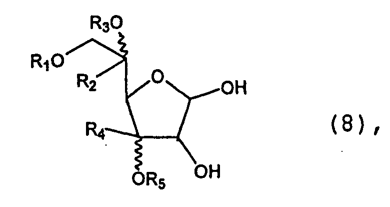 worin R₁, R₃ und R₅ eine geeignete Hydroxyschutzgruppe, wie sie im Stand der Technik etwa aus Protective Groups in Organic Synthesis (Greene, Wuts), 3. Auflage, John Wiley & Sons, Inc., Seiten 17 bis 245, bekannt sind, insbesondere eine Benzoyl- oder Acetylgruppe, bedeuten, R₂ und R₄ jeweils Wasserstoff oder Alkyl mit 1 bis 6 C-Atomen bedeuten, und die gewellte Linie jeweils beide mögliche Konfigurationen von -OR₃ bzw. -OR₅ in Bezug auf den Grundkörper bedeutet, in den Positionen 1 und 2 mit an sich bekannten, zur Aktivierung der Lactolgruppe geeigneten Schutzgruppen, vorzugsweise mit Phenoxyacetyl- Acetyl- oder Benzoylgruppen, versehen wird, wodurch die Lactolgruppe der so erhaltenen Verbindung der allgemeinen Formel 9 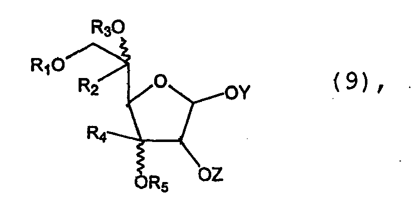 worin R₁-R₅ die in Formel 8 angegebene Bedeutung haben und X und Y jeweils eine an sich bekannte, zur Aktivierung der Lactolgruppe geeigneten Schutzgruppe, vorzugsweise eine Phenoxyacetyl-, Acetyl- oder Benzoylgruppe, darstellen, für die Nukleosidierung aktiviert wird. Anschließend wird die Verbindung der allgemeinen Formel 9 mit einem geschützten Cytosinderivat der allgemeinen Formel 4 wie eingangs beschrieben unter Erhalt einer Verbindung der allgemeinen Formel 10 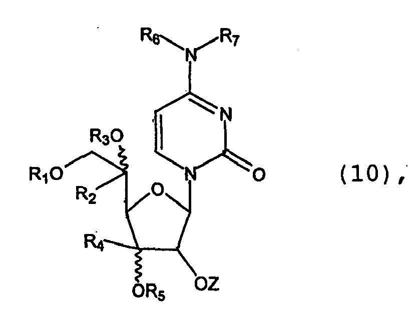 umgesetzt, wobei die jeweiligen Substituenten die in Formel 9 bzw. Formel 4 angegebenen Bedeutungen haben. Die Reaktionsdurchführung kann in Analogie zu der bereits oben beschriebenen Nukleosidierung erfolgen. Ein essentieller Schritt besteht in der Folge in der selektiven Abspaltung der Schutz- bzw. Aktivierungsgruppe in Position 2 der Verbindung der allgemeinen Formel 10, welche vorzugsweise als Hydrazinolyse unter Erhalt einer Verbindung der allgemeinen Formel 11 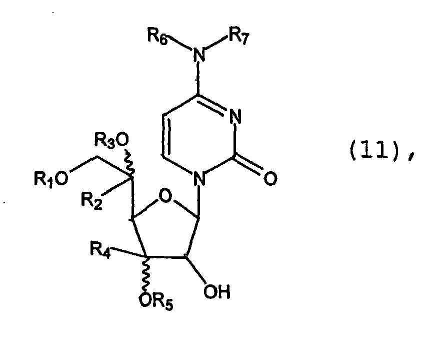 durchgeführt wird, worin die Substituenten die in Formel 10 angegebenen Bedeutungen haben. Durch Oxidation der Hydroxylgruppe in Position 2 der Verbindung der allgemeinen Formel 11, welche nach verschiedenen bekannten Verfahren, wie Oxidation mit Chromverbindungen oder Swern-Reaktion, vorzugsweise jedoch mit Hilfe des Katalysators TEMPO durchgeführt werden kann, wird eine Verbindung der allgemeinen Formel 12 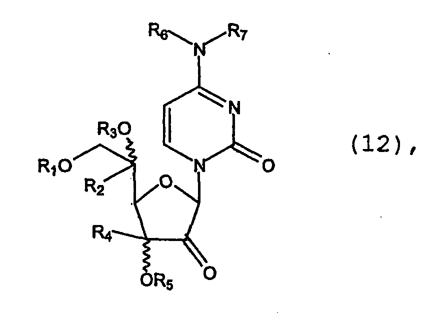 erhalten, worin die Substituenten die in Formel 10 angegebenen Bedeutungen haben. Anschließend erfolgt schließlich die Difluorierung der Verbindung der allgemeinen Formel 12, beispielsweise mit Hilfe von DAST zur Verbindung der allgemeinen Formel 2, 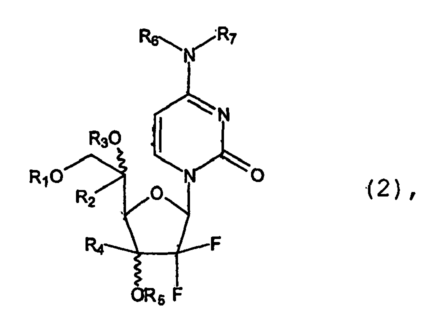 worin die Substituenten, gegebenenfalls nach Entfernen der Schutzgruppen an den Hydroxylgruppen des Zuckerteils und am Cytosinrest, die in Formel 2 eingangs angegebenen Bedeutungen haben. Es versteht sich, dass auch die so erhaltene Verbindung der allgemeinen Formel 2 wie bereits vorstehend beschrieben in Gemcitabin der Formel 1 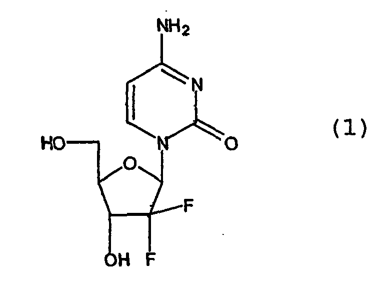 umgewandelt werden kann, wonach durch Umkristallisation Gemcitabin pharmazeutischer Qualität erhalten wird.

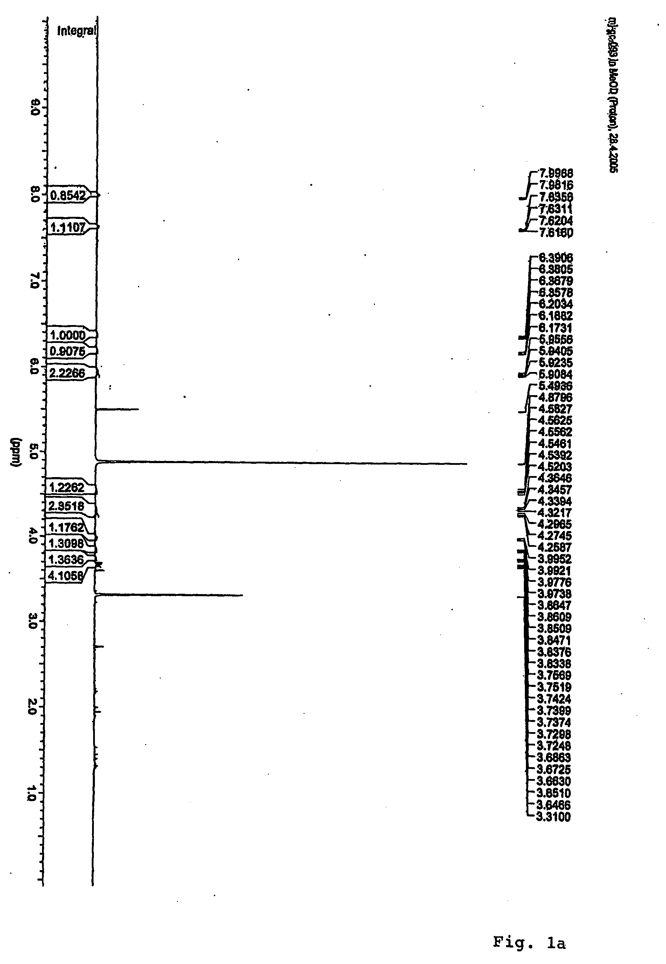

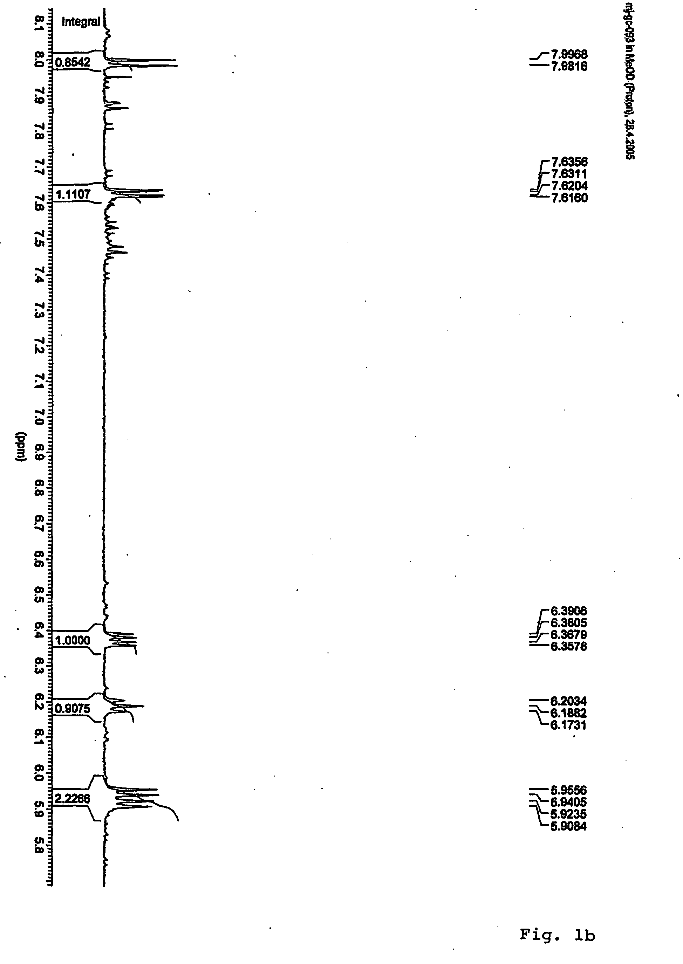

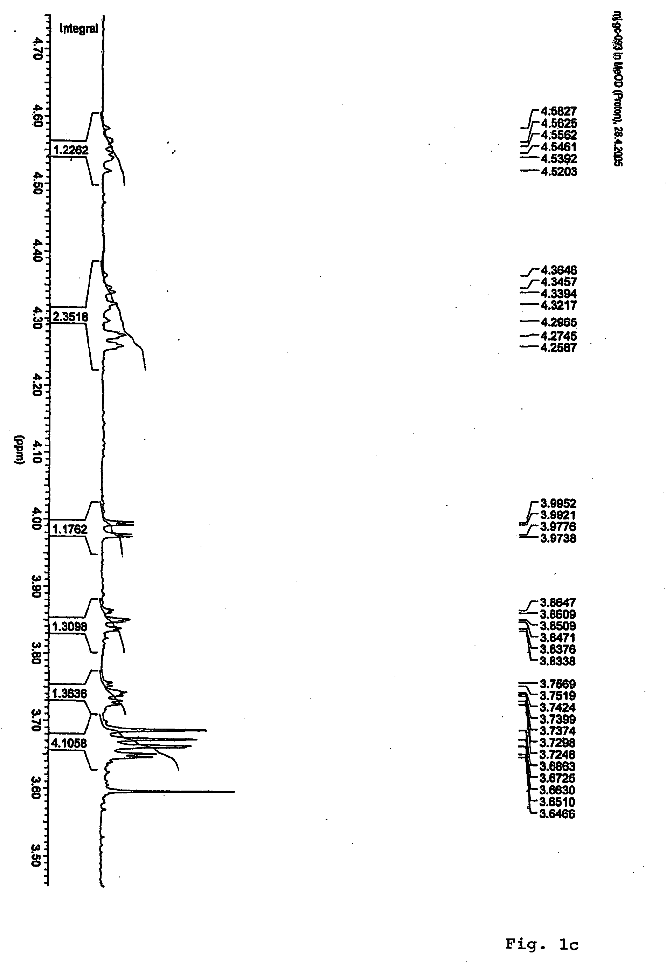

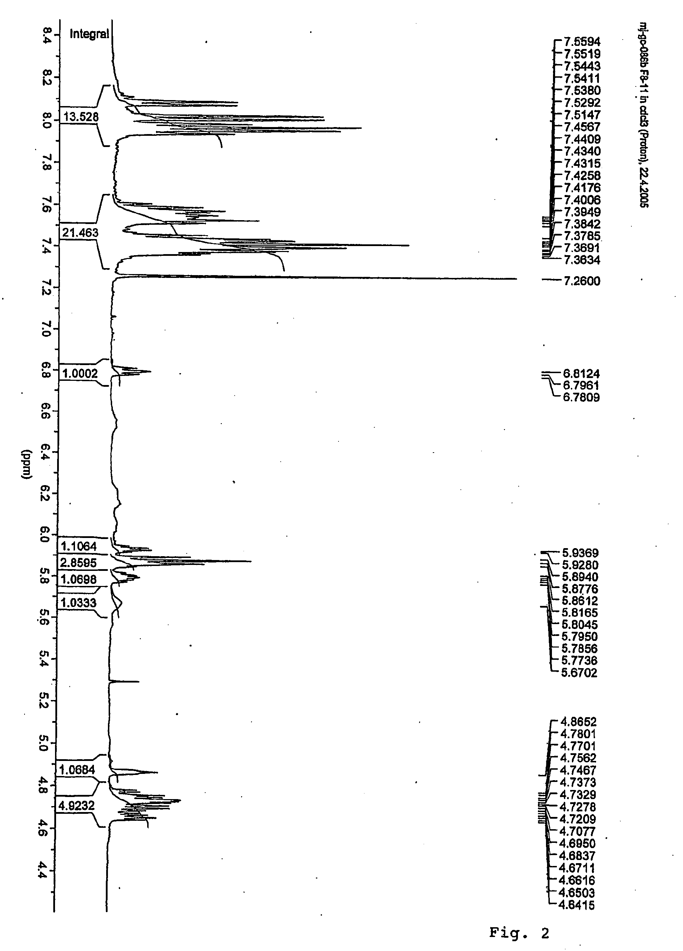

Die vorliegende Erfindung wird nun anhand der nachfolgenden Beispiele näher erläutert. In den beiliegenden Figuren stellen Fig. 1a, 1b und 1c die ¹H-NMR-Spektren des Anomerengemisches der Verbindung (XIII), eines Homogemcitabins der allgemeinen Formel 2 worin R₁ bis R₆ Wasserstoff bedeuten, und Fig. 2 das ¹H-NMR-Spektrum des Anomerengemisches der Verbindung (XII), einer Verbindung der allgemeinen Formel 2 worin R₁, R₃ und R₅ eine Benzoylgruppe bedeuten und R₂ und R₄ jeweils Wasserstoff sind, dar.

### Beispiel 1:

### 2,2-Dimethyl-1,3-dioxolan-4,5-dimethanol-4-benzoat (V)

Zu einer Lösung von 17 g 2,2-Dimethyl-1,3-dioxolan-4,5-dimethanol in 34 ml Pyridin werden bei 0°C 14,76 g Benzoylchlorid rasch zugesetzt. Nach 2 stündigem Rühren unter Kühlung wird das Reaktionsgemisch mit MTBE und H₂O versetzt. Die Phasen werden getrennt, die wässrige Phase wird mit MTBE extrahiert. Die vereinigten organischen Phasen werden mit halbgesättigter NaHCO₃-Lösung gewaschen, getrocknet und eingedampft. Der Rückstand wird mittels VFC (vacuum flash chromatographie) über Kieselgel mit Toluol/EtOAc (10+1), dann (1+1) aufgetrennt. Es werden 13,7 g Monobenzoat und 10,5 g Dibenzoat erhalten.

2,2-Dimethyl-1,3-dioxolan-4,5-dimethanol-4-monobenzoat: Bp: 113-117°C (0,05 mm Hg).
¹H-NMR: (CDCl₃) : δ(ppm) = 8,05-7,38 (m, 5H, Ar-H), 4,55-4,37 (m, 2H, CH₂), 4,29-4,20 (m, 1H, H-3), 4,08-4,00(m, 1H, H-2), 3,84-3,74 (dd, 2H, CH₂OH) 2,47-2,32 (s, 1H, OH), 1,43 (s, 6H, 2 x CH₃).
2,2-Dimethyl-1,3-dioxolan-4,5-dimethanol-4,5-dibenzoat; Fp: 85-86° C; ¹H-NMR: (CDCl₃) : δ (ppm) : 8,08-7,39 (m, 10 H, Ar-H), 4,63-4,51 (m, 4 H, 2 x CH₂), 4,38-4,28 (m, 2 H, H-2, H-3), 1,48 (s, 6 H, 2 x CH₃) ;

### Beispiel 2:

### 5-Benzoxymethyl-2,2-dimethyl-1,3-dioxolan-4-carbaldehyd (VI)

### a. mittels Swern-Oxidation

Zu einer Lösung von 1,86 g Oxalylchlorid in 13 ml wasserfreiem Dichlormethan werden bei -70°C 1,76 g DMSO in 10 ml Dichlormethan so zugesetzt, dass die Temperatur unter -60°C bleibt. Nach 15 min wird eine Lösung von 8,3 g 2,2-Dimethyl-1,3-dioxolan-4,5-dimethanol-4-benzoat (V) in 25 ml wasserfreiem Dichlormethan bei derselben Temperatur zugegeben und die Lösung 1 Stunde bei -70°C gerührt. Es werden 8,52 ml Triethylamin/Dichlormethan (1:1) bei einer Temperatur unter -60°C zugegeben. Nach 15 min wird die Kühlung entfernt und das Reaktionsgemisch nach Erreichen der Raumtemperatur mit 40 ml H₂O versetzt. Die Phasen werden getrennt, die wässrige Phase wird mit Dichlormethan extrahiert. Die vereinigten organischen Phasen werden mit 1N HCl, 5% NaHCO₃-Lösung und gesättigter NaCl-Lösung gewaschen, getrocknet und eingedampft. Es werden 2,73 g (91,7%) 5-Benzoxymethyl-2,2-dimethyl-1,3-dioxolan-4-carbaldehyd als gelbes Öl erhalten.
Bp.: 107-109°C (0,05 mm Hg).
¹H-NMR: (CDCl₃) : δ(ppm) = 9,83-9,82 (s, 1H, HC=O), 8,06-7,26 (m, 5H, Ar-H), 4,60-4,56 (m, 2H, CH₂), 4,47-4,34 (m, 1H, CH), 4,33 (m, 1H, CH), 1,51-1,49 (s, 3H, CH₃), 1,45-142 (s, 3H, CH₃).

### b. mittels Chromoxidation

Eine Lösung von 1,5 g 2,2-Dimethyl-1,3-dioxolan-4,5-dimethanol-4-benzoat (V) in trockenem Dichlormethan wird mit 5,6 g Pyridin und 2,6 g Chromtrioxid versetzt. Nach 3 Stunden rühren bei RT wird das Reaktionsgemisch mit MTBE verdünnt, über Celite filtriert, eingedampft und getrocknet. Es werden 0, 7 g 5-Benzoxymethyl-2,2-dimethyl-1,3-dioxolan-4-carbaldehyd als hellgelbes öl erhalten.

### Beispiel 3:

### 5-Benzoxymethyl-α,α-difluor-β-hydroxy-2,2-dimethyl-4-[1,3]-dioxolanpropansäureethylester (VII)

Zu einer Lösung von 18,9 g 5-Benzoxymethyl-2,2-dimethyl-1,3-dioxolan-4-carbaldehyd (VI) und 13 g Bromdifluoressigsäureethylester in 240 ml wasserfreiem THF/Diethylether (1:1) wird 4,2 g aktivierter Zinkstaub zugegeben. Das Reaktionsgemisch wird im Ultraschallbad 4 Stunden auf Rückflusstemperatur erhitzt. Anschließend werden 240 ml 0,5N HCl zugesetzt und die wässrige Phase dreimal mit MTBE extrahiert. Die gesammelten organischen Phasen werden mit 5% NaHCO₃-Lsg. gewaschen, über Na₂SO₄ getrocknet, filtriert und eingedampft. Es werden 7,85 g öliges Produkt erhalten.
¹H-NMR: (CDCl₃) : δ (ppm) = 8,07-7,42 (m, 5H, Ar-H), 4,69-4,67/4,50-4,62 (m, 2H, CH₂), 4, 44-4, 41 (m, 1H, CH), 4,40-4,23 (m, 3H, CH₂, CH), 4,09-4,05 (t, 1H, CH), 1,40-1,38 (d, 6H, 2 x CH₃), 1,37-134 (m, 3H, CH₃) .

### Beispiel 4:

### 1-(4,4-Difluoro-tetrahydro-3-hydroxy-5-oxo-2-furyl)-1,2-ethandiol-2-benzoat (VIII)

Eine Lösung von 5,9 g 5-Benzoxymethyl-α,α-difluor-β-hydroxy-2,2-dimethyl-4-[1,3]-dioxolanpropansäureethylester (VII) in 30 ml Acetonitril wird mit 1,5 g H₂O und 0.4 g TFA versetzt und 3 Stunden unter Rückfluss erhitzt. Das Acetonitril wird abdestilliert und Toluol zugegeben. Es wird 14 h unter Rückfluss und Wasserabscheidung erhitzt. Das Lösungsmittel wird im Vakuum abdestilliert und der Rückstand aus Dichlormethan umkristallisiert. Es werden 2,4 g farblose Kristalle erhalten (
Fp. 110°C) . ¹H-NMR: (d4-MeOH): δ (ppm) = 8,08-7,48 (m, 5H, Ar-H), 4,61-4,54 (m, 1H, H-3), 4,50-4,42 (m, 3H, 2 x H-6, H-4), 4,22-4,19 (m, 1H, H-5).

### Beispiel 5:

### 1-(4,4-Difluoro-tetrahydro-3-hydroxy-5-oxo-2-furyl)-1,2-ethandiol-1,2,3'-tribenzoat (IX)

Zu einer Lösung von 1,95 g 1-(4,4-Difluoro-tetrahydro-3-hydroxy-5-oxo-2-furyl)-1,2-ethandiol-2-benzoat (VIII), 1,95 g Pyridin und 0,16 g Dimethylaminopyridin (DMAP) in Ethylacetat werden 2,13 g Benzoylchlorid in Ethylacetat bei Rückflusstemperatur zugetropft. Nach weiteren 3 Stunden Erhitzen unter Rückfluss wird das Reaktionsgemisch auf RT abgekühlt, mit Ethylacetat verdünnt und mit Wasser extrahiert. Die wässrige Phase wird mit Ethylacetat rückextrahiert, die vereinigten organischen Phasen werden nacheinander mit verdünnter 1N HCl-Lösung und mit gesättigter Natriumbicarbonatlösung extrahiert, getrocknet und eingedampft. Es werden 3 g Öl erhalten.
¹H-NMR: (CDCl₃) : δ (ppm) = 8,04-7,39 (m, 15H, 3 x Ar-H), 5, 99-5, 98 (m, 1H, H-5), 5,76-5,73 (m, 1H, H-3), 5,15-5,14 (s, 1H, H-4), 4,79-4,76 (m, 2H, 2 x H-6).

### Beispiel 6:

### 5-{[1,2-Bis(benzoyloxy)]ethyl}-3,3-difluor-tetrahydrofuran-2,4-diol-4-benzoat (X)

Zu einer Lösung von 3 g 1-(4,4-Difluoro-tetrahydro-3-hydroxy-5-oxo-2-furyl)-1,2-ethandiol-1,2,3'-tribenzoat (IX) in 10 ml THF und 40 ml Diethlyether werden bei 0°C 2,1 g Lithiumaluminiumtritertiärbutylaluminiumhydrid (1 M Lösung in THF) zugetropft. Nach zweistündigem Rühren wird unter Kühlung mit 1N HCl angesäuert. Die Lösung wird mit MTBE verdünnt und die Phasen werden getrennt. Die wässrige Phase wird nochmals mit MTBE extrahiert, die vereinigten organischen Phasen werden mit 5% NaHCO₃ Lösung neutralisiert, getrocknet und eingedampft. Es werden 2,71 g hellgelbes Öl erhalten.
¹H-NMR: (CDCl₃) : δ (ppm) = 7,61-7,26 (m, 15H, 3 x Ar-H), 6,01-4,95/5,47-5,43 (m, 1H, H-3), 5,93-5,91/5,70-5,67 (m, 1H, H-5), 5, 42/5, 28 (d, 1H, H-1), 4,79-4,58 (m, 3H, H-4, 2 x H-6).

### Beispiel 7:

### 5-{[1,2-Bis(benzoyloxy)]ethyl}-3,3-difluor-tetrahydrofuran-2,4-diol-2-methansulfonat-4-benzoat (XI)

Eine Lösung von 3,3 g 5-{[1,2-Bis(benzoyloxy)]ethyl}-3,3-difluor-tetrahydrofuran-2,4-diol-4-benzoat (X) in wasserfreiem Dichlormethan wird bei 0°C mit 0,98 g Triethylamin und anschließend mit 0,89 g Methansulfonychlorid langsam versetzt und für dreißig Minuten unter Kühlung und dann 2 Stunden bei Raumtemperatur gerührt. Das Reaktionsgemisch wird mit Dichlormethan verdünnt und nacheinander mit 1 N HCl und 5% NaHCO₃ -Lösung extrahiert. Die organische Phase wird getrocknet und eingedampft. Es werden 3,44 g öliges Produkt erhalten.
¹H-NMR: (CDCl₃) : δ (ppm) = 8,14-7,34 (m, 15H, 3 x Ar-H), 6,19-6,18/6,08-6,06 (d, 1H, H-1), 5,99-5,79 (m, 2H, H-3, H-5), 5,75-5,73 (q, 1H, H-1), 5,57-5,47 (m, 1H, H-3), 4,93-4,92/4,79-4,76 (m, 1H, H-4), 4,69/4,64 (m, 2H, 2 x H-6), 3,16/3,15 (s, 3H, CH₃).

### Beispiel 8:

### 4-Amino-1-{5-{[1,2-Bis(benzoyloxy)]ethyl}-4-(benzoyloxy)-3,3-difluor-tetrahydro-2-furanyl}-2(1H)-pyrimidinon (XII) und 4-Acetylamino-1-{5-{[1,2-Bis(benzoyloxy)]-ethyl}-4-(benzoyloxy)-3,3-difluor-tetrahydro-2-furanyl}-2(1H)-pyrimidinon (XIIa)

### a. Bis(trimethylsilyl)-N-acetylcytosin

Eine Suspension aus N-Acetylcytosin, Hexamethyldisilazan und Ammoniumsulfat wird 5 Stunden unter Rückfluss erhitzt. Anschließend wird überschüssiges Hexamethyldisilazan abdestilliert und das Rohprodukt destilliert. Es werden 0,7 g Produkt als hellgelbes Öl erhalten, Bp.: 150°C/0,16 mm Hg.

### b. Nukleosidierung

Eine Lösung von 0,54 g Bis(trimethylsilyl)-N-acetylcytosin in Dichlorethan wird mit 0,41 g Trifluormethansulfonsäuretrimethylsilylester 1 h bei Raumtemperatur gerührt, mit 0,72 g 5-{[1,2-Bis(benzoyloxy)]ethyl}-3,3-difluor-tetrahydrofuran-2,4-diol-2-methansulfonat-4-benzoat (XI) versetzt und 16 h unter Rückfluss erhitzt. Das Reaktionsgemisch wird mit Dichlorethan verdünnt und zunächst mit Wasser, anschließend mit 5%-Natriumbicarbonatlösung extrahiert. Die organische Phase wird getrocknet und eingedampft. Das Rohprodukt wird über VFC aufgetrennt. Es werden 48 mg 4-Acetylamino-1-{5-{[1,2-Bis(benzoyloxy)]-ethyl}-4-(benzoyloxy)-3,3-difluor-tetrahydro-2-furanyl}-2(*1H*)-pyrimidinon und 287 mg 4-Amino-1-{5-{[1,2-Bis(benzoyloxy)]ethyl}-4-(benzoyloxy)-3,3-difluor-tetrahydro-2-furanyl}-2(*1H*)-pyrimidinon als Anomerengemisch erhalten. Die Mischung kann auch ungetrennt in die nächste Reaktion eingesetzt werden.
¹H-NMR (Anomerengemisch v. XII): (CDCl₃): δ (ppm) = 8,10-7,85 (m, 6H, 3 x Benzoyl-H-2,6); 7,62-7,35 (m, 10H, Benzoyl-H-3,4,5, H-6), 6,77-6,59 (m, 1H, H-1'), 5,94-5,92/5,89-5,86 (m, 2H, H-5, H-5'),5,82-5,77/5,67 (m, 1H, H-3'), 4,86 (m, 1H, H-4'), 4,78-4,64 (m, 3H, H-4', 2 x H-6'), vgl. auch Fig. 1a, 1b und 1c.

### Beispiel 9:

### 4-Amino-1-{5-[(1,2-dihydroxy)]ethyl]-3,3-difluor-tetrahydro-4-hydroxy-2-furanyl}-2(1H)-pyrimidinon (XIII) und 4-Acetylamino-1-{5-[(1,2-dihydroxy)]ethyl]-3,3-difluor-tetrahydro-4-hydroxy-2-furanyl}-2(1H)-pyrimidinon (XIIIa)

87 mg einer Mischung von 4-Amino-1-{5-{[1,2-Bis(benzoyloxy)]ethyl}-4-(benzoyloxy)-3,3-difluor-tetrahydro-2-furanyl}-2(*1H*)-pyrimidinon (XII) und 4-Acetylamino-1-{5-{[1,2-Bis(benzoyloxy)]-ethyl}-4-(benzoyloxy)-3,3-difluor-tetrahydro-2-furanyl}-2(*1H*)-pyrimidinon (XIIa) werden mit 7n NH₃ in Methanol 16 Stunden bei RT gerührt und anschließend zur Trockene eingedampft. Der Rückstand wird in Wasser aufgenommen und mit Diethylether extrahiert. Die wässrige Phase wird im Vakuum eingedampft. Es werden 49 mg hellbraunes öl erhalten.
¹H-NMR (Anomerengemisch): (MeOD): δ (ppm) = 8, 00-7, 98/7, 63-7, 61 (m, 1H, H-6), 6, 39-6, 36/6, 20-6, 17 (m, 1H, H-1'), 5,96-5,90 (m, 1H, H-5), 4, 58-4, 52/4, 36-4, 30 (m, 1H, H-3'), 4,29-4,26/3,99-3,97 (m, 1H, H-4'), 3,86-3,83/3,76-3,73 (m, 1H, H-5'), 3,72-3,65 (m, 2H, 2 x H-6'), vgl. auch Fig. 2

### Beispiel 10:

### Gemcitabin x HCl

Zu einer Lösung von 0,08 g einer Mischung von 4-Amino-1-{5-[(1,2-dihydroxy)]ethyl]-3,3-difluor-tetrahydro-4-hydroxy-2-furanyl}-2(1H)-pyrimidinon (XIII) und 4-Acetylmino-1-{5 -[(1,2-dihydroxy)]ethyl]-3,3-difluor-tetrahydro-4-hydroxy-2-furanyl}-2(1H)-pyrimidinon (XIIIa) in Methanol wird unter Eiskühlung eine Lösung von 0,1 g Natriumperjodat in Wasser zugetropft und noch 15 min unter Eiskühlung, dann 1 h bei Raumtemperatur gerührt. Anschließend wird unter Eiskühlung 0,02 g Natriumborhydrid zugegeben und nach 15 min noch eine Stunde bei Raumtemperatur gerührt. Der Feststoff wird abfiltriert, das Filtrat wird mit 5n HCl in i-Propanol neutralisiert und zur Trockene eingedampft, in DCM/MeOH(4+1) aufgenommen und über Kieselgel filtriert. Es werden 0,06 g Gemcitabin x HCl erhalten. Zur weiteren Reinigung wird aus Aceton/Wasser umkristallisiert.
Fp: (271-76, dec.) ¹H-NMR: (MeOD): δ (ppm) = 8,09-8,07/7,68-7,85 (m, 1H, H-6), 6,35-6,32/6,22-6,19 (m, 1H, H-1'), 6,09-6,05 (m, 1H, H-5), 4,46-4,38/4,33-4,25 (m, 1H, H-3'), 3,96-3,93/3,81-3,78 (m, 1H, H-4'), 3,72-3,61 (m, 1H, H-5').
Es wird nun die alternative Synthese der Verbindung 4-Amino-1-{5-{[1,2-bis(benzoyloxy)]ethyl}-4-(benzoyloxy)-3,3-difluor-tetrahydro-2-furanyl}-2(*1H*)-pyrimidinon (XII), vgl. Beispiel 8, detailliert beschrieben:

### 1,2-Isopropyliden-3,5,6-Tribenzoyl-allofuranose (XV)

8,04 g 1,2-Isopropyliden-3-Benzoyl-allofuranose (XIV) werden mit 0,25 g DMAP in 90 ml Dichlormethan/Pyridin 2:1 v/v gelöst. Unter Argonatmosphäre werden 6,6 ml Benzoylchlorid zugetropft und 24 Stunden bei Raumtemperatur gerührt. Die Reaktion wird mit 20 ml MeOH gequencht. Die Mischung wird mit Wasser verdünnt. Die organische Phase wird 2 Mal mit 40 ml Wasser gewaschen über Na₂-SO₄ getrocknet und eingedampft. Der Rückstand wird in 60 ml Dichlormethan gelöst, 2 Mal mit 40 ml 1N H₂SO₄ und 2 Mal mit 40 ml gesättigter NaHCO₃ gewaschen. Die organische Phase wird mit Na₂SO₄ getrocknet und das Lösungsmittel wird abrotiert. Es werden 12,2 g Produkt als weißes Pulver erhalten.
¹H NMR: (CDCl₃) : δ (ppm) = 8,01-7,77 (m, 6H, 3 x Benzoyl-H-2,6); 7,35-7,20 (m, 9H, 3 x Benzoyl-H-3,4,5); 5,82 (d, 1H, H-1); 5,72 (m, 1H, H-2); 5,09 (m, 1H, H-3); 4,95 (m, 1H, H-5); 4,60-4,56 (m, 3H, 2x H-6, H-4); 1,49 und 1,25 (s, 6 H, 2x Isopropyliden-CH₃) .

### 3,5,6-Tribenzoyl-allofuranose (XVI)

### (Ketalspaltung)

1,34 g 1,2-Isopropyliden-3,5,6-Tribenzoyl-allofuranose (XV) (2,52 mmol) hergestellt gemäß dem obigen Beispiel werden in 10 ml 0,1 N HCl / Acetonitril gelöst und bei 50 ° C 2,5 Stunden lang gerührt. Das Lösungsmittel wird abgedampft, der Rückstand in Dichlormethan aufgenommen und 2x mit Wasser gewaschen. Die organische Phase wird über Na₂SO₄ getrocknet, abfiltriert und das Lösungsmittel abgedampft. Die erhaltene Mischung wird chromatographisch getrennt (Laufmittel DCM/EtOAc 3:1). Es werden 600 mg Produkt als weißen Feststoff erhalten.
¹H NMR: (CDCl₃) : δ (ppm) = 8,01-7,91 (m, 6H, 3 x Benzoyl-H-2, 6) ; 7,70-7,23 (m, 9H, 3 x Benzoyl-H-3,4,5); 5,70-5,61 (m, 1H, H-1); 5,56-5,45 (m, 2H, H-3, H-5); 4,71-4,38 (m, 3H, 2 x H-6, H-2); 4,36 (t, 1H, H-4).

### Beispiel 11:

### 4,5,6-Tribenzoyl-1,2-di(phenoxyacetyl)-allo-furanose (XVII)

Eine Lösung von 184 mg 3,5,6-Tribenzyolallofuranose (XVI) in 4 ml Pyridin wird bei 0° C unter Argonatmosphäre tropfenweise mit 127 mg Phenoxyacetylchlorid versetzt und eine Stunde lang gerührt. Die Reaktion wird mit 0,5 ml Methanol gequencht. Nach Zusatz von 10 ml Toluol wird im Vakuum eingedampft. Der Rückstand wird mit Petrolether/Ethylacetat 3:1 - 1:1 über 15 g Kielselgel chromatographiert. Es werden 225 mg gelbliches Öl erhalten.
¹H NMR: (CDCl₃): δ (ppm) = 8,03-7,96 (m, 6H, 3 x Benzoyl-H-2,6), 7,60-6,40 (m, 19H, 3 x Benzoyl-H-3,4,5, 2 x Phenoxyacetyl-H-2,3,4,5,6), 6,77 (m, 1 H, H-1'), 5,92-5,70 (3H, m, H-2', H-3', H-5'); 4,70-4,32 (m, 7H, H-4', 2 x Phenoxyacetyl-CH₂, 2 x H-6').

### Beispiel 12:

### 1-[3,5,6-Tribenzoyl-2-Phenoxyacetyl-allofuranosyl]-N-acetyl-cytosin (XVIII) (Nukleosidierung)

Zur Herstellung eines geschützten Cytosinderivats wird eine Suspension von 30,5 mg N-Acetylcytosin in 3 ml wasserfreiem Dichlorethan mit 152,5 mg Bistrimethylsilylacetamid versetzt und unter Argonatmosphäre unter Rühren bis zur vollständigen Klärung der Lösung unter Rückfluß erhitzt. Anschließend wird eine Suspension von 200 mg 3,5,6-Tribenzoyl-1,2-di(phenoxyacetyl)-allofuranose (XVII) in 3 ml trockenem Dichlorethan zu der auf 50°C gekühlten Lösung zugetropft. Nach tropfenweiser Zugabe von 100 mg Trifluormethylsilyltrifluormethansulfonat wird 16 h bei 80°C gerührt. Das Reaktionsgemisch wird zwischen Dichlormethan und gesättigter wäßriger NaHCO₃-Lösung verteilt. Danach wird die organische Phase mehrmals mit Wasser und NaCl-Lösung gewaschen. Die vereinigten organischen Phasen werden über Na₂SO₄ getrocknet, filtriert und am Vakuum eingeengt. Das Reaktionsgemisch wird mittels Vakuum Flash Chromatographie (DCM-MeOH 9:1) weiter gereinigt. Es werden 185 mg des Anomerengemisches erhalten. Das Produkt kann ohne weitere Reinigung in die nächste Reaktion eingesetzt werden.
¹H NMR: (CDCl₃) : δ (ppm) = 9,76 (s, 1H, NH); 8,13-7, 85 (m, 6H, 3 x Benzoyl-H-2,6); 7,58-6,89 (m, 13H, 3 x Benzoyl-H-3,4,5; Phenoxyacetyl-H-2,4,6, H-6); 6,86 (m, 1H, H-1'); 6,74 (m, 2H, Phenoxyacetyl-H-3,5); 6,15-5,80 (m, 4H, H-5, H-5', H-3', H-2'); 4,91-4,43 (m, 5H, Phenoxyacetyl-CH₂, H-4', 2 x H-6'); 2,42 (s, 3H, N-Acetyl-CH₃).

### Beispiel 13:

### 1-[3,5,6-Tribenzoyl-allofuranosyl]-cytosin (XIX) (selektive Verseifung)

Zu einer Lösung von 80 mg 1-[3,5,6-Tribenzoyl-2-Phenoxyacetyl-allofuranosyl]-N-acetyl-cytosin (XVIII) in 1,5 ml Eisessig-Pyridin Mischung (1:4 v/v) werden unter einer Argonatmosphäre 17 mg Hydrazinmonohydrat als 5 prozentige Lösung in Eisessig-Pyridin zugetropft. Die Lösung wird 15 Stunden bei 70-75°C gerührt. Anschließend werden 2ml Aceton zugetropft, das Reaktionsgemisch wird mit Dichlormethan verdünnt und mehrmals mit Wasser gewaschen. Die vereinigten organischen Phasen werden über Natriumsulfat getrocknet, filtriert und eingedampft. Nach Vakuum Flash Chromatographie (Eluens: Dichlormethan mit 1% bis 4% MeOH) werden 53 mg Produkt als Öl erhalten.
¹H NMR: (CDCl₃) : δ (ppm) = 8,14-7,96 (m, 6H, 3 x Benzoyl-H-2,6), 7,64-7,26 (m, 10H, 3 x Benzoyl-H-3,4,5, H-6); 6,91-6,77 (m, 1H, H-1'); 5,89-5,61 (m, 3H, H-5, H-3', H-5'); 4,97-4,43 (m, 4H, H-2 ', H-4', 2 x H-6').

### Beispiel 14:

### 4-Amino-1-{5-{[1,2-bis(benzoyloxy)]ethyl}-4-(benzoyloxy)-tetrahydro-3-oxo-2-furanyl}-2(1H)-pyrimidinon (XX) (Oxidation)

80 mg 1-[3,5,6-Tribenzoyl-allofuranosyl]-cytosin (XIX) werden mit 1,2 mg TEMPO (2,2,6,6-Tetramethylpiperidin-1-oxyl) vermengt und in 5 ml Dichlormethan gelöst. Die Lösung wird am Eis-Wasser-Bad auf 0-5°C gekühlt. 1,8 mg Kaliumbromid werden in 0,25 ml H₂O gelöst und zur Mischung zugetropft. NaOCl wird mit NaHCO₃ auf pH 9,5 eingestellt und 0,26 ml hievon werden langsam unter Temperaturkontrolle zugetropft. Es wird weitere 10 Minuten am Eis-Wasser-Bad gerührt. Durch DC-Kontrolle (DCM/MeOH 9:1) wird die vollständige Umsetzung beobachtet. Die organische Phase wird eingedampft, es werden 70 mg Produkt als Öl erhalten.
¹H NMR: (CDCl₃) : δ (ppm) = 8,02-7,82 (m, 6H, 3 x Benzoyl-H-2,6); 7,83-7,30 (m, 10H, 3 x Benzoyl-3,4,5, H-6, H-5); 6,90-6,59 (m, 1H, H-1'); 6,08-5,56 (m, 3H, H-5, H-5', H-3'); 4,87-4,71 (m, 3H, 2x H-6', H-4').

### Beispiel 15:

### 4-Amino-1-{5-{[1,2-bis (benzoyloxy)]ethyl}-4-(benzoyloxy)-3,3-difluor-tetrahydro-2-furanyl)-2(1H)-pyrimidinon (XII) (Fluorierung)

100 mg 4-Amino-1-{5-{[1,2-bis(benzoyloxy)]ethyl}-4-(benzoyloxy)-tetrahydro-3-oxo-2-furanyl}-2(*1H*)-pyrimidinon (XX) werden in 1 ml Dichlormethan gelöst. Es wird bei Raumtemperatur gerührt, anschließend werden 20 mg DAST (Diethylaminoschwefeltrichlorid) zugetropft. Nach vollständiger Zugabe wird Pyridin-HF (ca. 30 µl) zugesetzt. Die Mischung wird 48 Stunden lang bei Raumtemperatur gerührt, wonach 39 mg Produkt als Öl erhalten werden.
Das ¹H NMR (CDCl₃) der gemäß Beispiel 15 hergestellten Verbindung (XII) entsprach jenem der gemäß Beispiel 8 hergestellten Verbindung (XII), vgl. auch Fig. 2.

## Patentansprüche

1. Verbindungen der allgemeinen Formel 2, 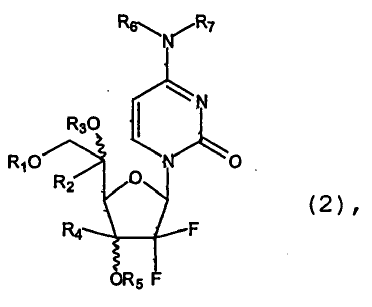 worin R₁, R₃ und R₅ Wasserstoff oder eine geeignete Hydroxyschutzgruppe, insbesondere eine Benzoylgruppe bedeuten, R₂ und R₄ jeweils Wasserstoff oder Alkyl mit 1 bis 6 C-Atomen bedeuten, und R₆ und R₇ jeweils Wasserstoff oder eine geeignete Aminoschutzgruppe bedeuten und die gewellte Linie jeweils beide mögliche Konfigurationen von -OR₃ bzw. -OR₅ in Bezug auf den Grundkörper bedeutet.

2. Verbindung gemäß Anspruch 1, **dadurch gekennzeichnet, dass** R₁, R₃ und R₅ Wasserstoff oder eine Benzoylgruppe bedeuten, R₂ und R₄ jeweils Wasserstoff bedeuten, und R₆ und R₇ jeweils Wasserstoff, Acetyl, Alkylsilanyl oder Arylalkylsilanyl mit 1 bis 6 C-Atomen im Alkylteil, bedeuten.

3. Verbindung gemäß Anspruch 1 oder 2, **dadurch gekennzeichnet, dass** R₁ bis R₇ jeweils Wasserstoff bedeuten.

4. Verfahren zur Herstellung von Verbindungen der allgemeinen Formel 2 gemäß einem der Ansprüche 1 bis 3, **dadurch gekennzeichnet, dass** eine Verbindung der allgemeinen Formel 3 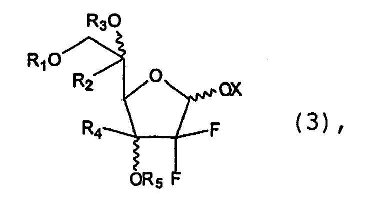 worin R₁ bis R₅ die in Anspruch 1 angegebene Bedeutung haben, X Wasserstoff oder eine an sich bekannte Aktivierungsgruppe darstellt und die gewellte Linie jeweils beide mögliche Konfigurationen von OX, -OR₃ bzw. -OR₅ in Bezug auf den Grundkörper bedeutet,
mit einem mit Schutzgruppen versehenen Cytosin der allgemeinen Formel 4 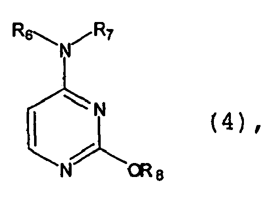 in welcher R₆ und R₇ die in Anspruch 1 angegebenen Bedeutungen haben und R₈ eine geeignete Abgangsgruppe ist,
umgesetzt wird,
wobei anschließend gegebenenfalls allfällige noch vorhandene Schutzgruppen abgespalten werden, um Verbindungen der allgemeinen Formel (2) zu erhalten, worin R₁ bis R₇ jeweils Wasserstoff bedeuten.

5. Verfahren nach Anspruch 4, **dadurch gekennzeichnet, dass** X einen Alkylsulfonylrest mit 1 bis 6 C-Atomen im Alkylteil bedeutet und mindestens eines von R₆ und R₇ Trialkylsilanyl oder Triarylalkylsilanyl mit jeweils 1 bis 6 C-Atomen im Alkylteil bedeutet.

6. Verfahren nach Anspruch 4 oder 5, **dadurch gekennzeichnet, dass** R₈ gleich mit R₆ und R₇ ist.

7. Verbindungen der allgemeinen Formel 3 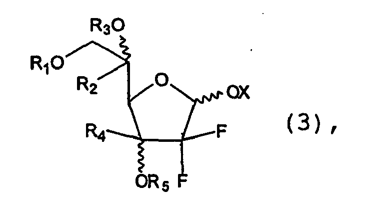 worin R₁ bis R₅ die in Anspruch 1 angegebene Bedeutung haben, X Wasserstoff oder eine an sich bekannte Aktivierungsgruppe darstellt und die gewellte Linie jeweils beide mögliche Konfigurationen von OX, -OR₃ bzw. -OR₅ in Bezug auf den Grundkörper bedeutet, oder die gewellte Linie zusammen mit OX eine Ketogruppe darstellt.

8. Verbindung nach Anspruch 7, **dadurch gekennzeichnet, dass** R₁ eine geeignete Hydroxyschutzgruppe bedeutet, R₂ bis R₅ Wasserstoff darstellen und die gewellte Linie zusammen mit OX eine Ketogruppe darstellt.

9. Verbindung nach Anspruch 8, **dadurch gekennzeichnet, dass** R₁ eine Benzoylgruppe bedeutet.

10. Verbindung nach Anspruch 7, **dadurch gekennzeichnet, dass** R₁, R₃ und R₅ eine geeignete Hydroxyschutzgruppe bedeuten und die gewellte Linie zusammen mit OX eine Ketogruppe darstellt.

11. Verbindung nach Anspruch 7, **dadurch gekennzeichnet, dass** R₁, R₃ und R₅ eine geeignete Hydroxyschutzgruppe bedeuten und X Wasserstoff darstellt.

12. Verbindung nach Anspruch 11, **dadurch gekennzeichnet, dass** R₁, R₃ und R₅ eine Benzoylgruppe bedeuten.

13. Verbindung nach Anspruch 7, **dadurch gekennzeichnet, dass** R₁, R₃ und R₅ eine geeignete Hydroxyschutzgruppe bedeuten und X eine Aktivierungsgruppe darstellt.

14. Verbindung nach Anspruch 13, **dadurch gekennzeichnet, dass** R₁, R₃ und R₅ eine Benzoylgruppe bedeuten und X einen Alkylsulfonylrest mit 1 bis 6 C-Atomen im Alkylteil darstellt.

15. Verfahren zur Herstellung von Verbindungen der allgemeinen Formel 2 gemäß einem der Ansprüche 1 bis 3, **dadurch gekennzeichnet, dass** eine Verbindung der allgemeinen Formel 12 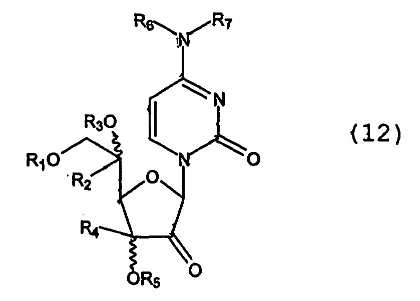 worin R₁ bis R₇ die in Anspruch 1 angegebene Bedeutung haben, und die gewellte Linie jeweils beide mögliche Konfigurationen von OX, -OR₃ bzw. -OR₅ in Bezug auf den Grundkörper bedeutet,
mit einem geeigneten Fluorierungsmittel fluoriert wird, wobei anschließend gegebenenfalls allfällige noch vorhandene Schutzgruppen abgespalten werden, um Verbindungen der allgemeinen Formel (2) zu erhalten, worin R₁ bis R₇ jeweils Wasserstoff bedeuten.

16. Verfahren nach Anspruch 15, **dadurch gekennzeichnet, dass** die Fluorierung mit DAST (Diethylaminoschwefeltrichloid) in Kombination mit HF erfolgt.

17. Verfahren nach einem der Ansprüche 15 und 16, **dadurch gekennzeichnet, dass** R₁, R₃ und R₅ eine Benzoylgruppe bedeuten und R₂, R₄, R₆ und R₇ jeweils Wasserstoff bedeuten.

18. Verbindung der allgemeinen Formel 12 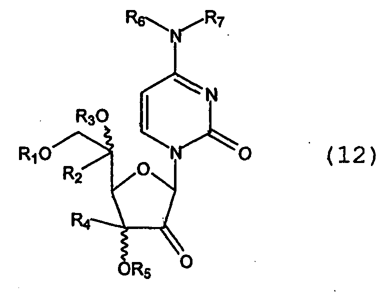 worin R₁ bis R₇ die in Anspruch 1 angegebene Bedeutung haben, und die gewellte Linie jeweils beide mögliche Konfigurationen von OX, -OR₃ bzw. -OR₅ in Bezug auf den Grundkörper bedeutet.

19. Verbindung nach Anspruch 18, **dadurch gekennzeichnet, dass** R₁, R₃ und R₅ eine Benzoylgruppe bedeuten und R₂, R₄, R₆ und R₇ jeweils Wasserstoff bedeuten und mindestens eines von R₆ und R₇ Wasserstoff, Trialkylsilanyl oder Triarylalkylsilanyl mit jeweils 1 bis 6 C-Atomen im Alkylteil bedeutet.

20. Verbindung nach Anspruch 19, **dadurch gekennzeichnet, dass** R₆ und R₇ beide Wasserstoff bedeuten.

21. Verfahren zur Herstellung von Gemcitabin, **dadurch gekennzeichnet, dass** eine Verbindung nach einem der Ansprüche 1 bis 3 gegebenenfalls entschützt und einer an sich bekannten Glykolspaltung zum Aldehyd der allgemeinen Formel 7 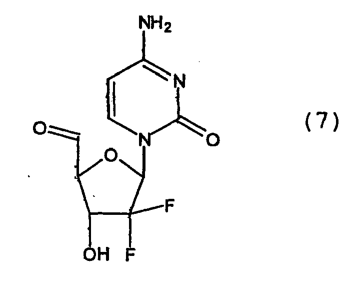 unterzogen wird, wonach die Aldehydgruppe des Aldehyds 7 mit einem komplexen Hydrid reduziert wird, wodurch Gemcitabin der Formel 1 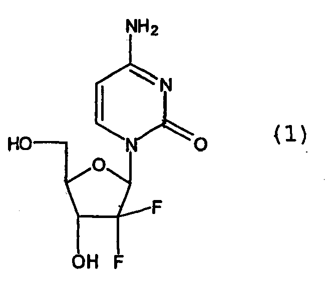 erhalten wird.

22. Verfahren nach Anspruch 21, **dadurch gekennzeichnet, dass** die Glykolspaltung mit einem Perjodat durchgeführt wird.

23. Verfahren nach einem der Ansprüche 21 oder 22, **dadurch gekennzeichnet, dass** mit Natriumborhydrid reduziert wird.

24. Verfahren nach einem der Ansprüche 21 bis 23, **dadurch gekennzeichnet, dass** in der Glykolspaltung das reine β-Anomere von Verbindungen der allgemeinen Formel 2, worin R₁ bis R₇ jeweils Wasserstoff bedeuten, eingesetzt wird.

25. Verfahren nach einem der Ansprüche 21 bis 23, **dadurch gekennzeichnet, dass** in der Glykolspaltung ein Gemisch des α- und β-Anomeren von Verbindungen der allgemeinen Formel 2, worin R₁ bis R₇ jeweils Wasserstoff bedeuten, eingesetzt wird.

26. Verfahren nach einem der Ansprüche 21 bis 25, **dadurch gekennzeichnet, dass** die Reduktion in einer Eintopfreaktion im Anschluss an die Glykolspaltung durchgeführt wird.

27. Verwendung einer Verbindung der allgemeinen Formel 2, 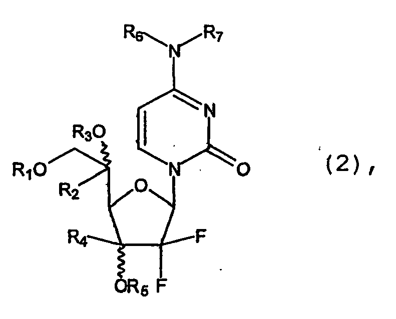 worin R₁, R₃ und R₅ Wasserstoff bedeuten, R₂ und R₄ jeweils Wasserstoff oder Alkyl mit 1 bis 6 C-Atomen bedeuten, und R₆ und R₇ jeweils Wasserstoff oder Acetyl bedeuten und die gewellte Linie jeweils beide mögliche Konfigurationen von -OR₃ bzw. -OR₅ in Bezug auf den Grundkörper bedeutet, zur Herstellung eines Arzneimittels zur Behandlung proliferativer Erkrankungen, insbesondere zur Behandlung von NSCLC (non-small cell lung cancer, Nicht-kleinzelliges Lungenkarzinom), Mammakarzinom, Ovarialkarzinom, Pankreaskarzinom und Harnblasenkarzinom, alleine oder in Kombination mit anderen Wirkstoffen/Medikamenten.

28. Verwendung einer Verbindung der allgemeinen Formel 2, 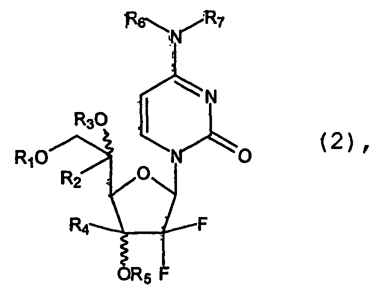 worin R₁ bis R₇ jeweils Wasserstoff bedeuten und die gewellte Linie jeweils beide mögliche Konfigurationen von -OR₃ bzw. -OR₅ in Bezug auf den Grundkörper bedeutet, zur Herstellung eines Arzneimittels zur Behandlung proliferativer Erkrankungen, insbesondere zur Behandlung von NSCLC (non-small cell lung cancer, Nicht-kleinzelliges Lungenkarzinom), Mammakarzinom, Ovarialkarzinom, Pankreaskarzinom und Harnblasenkarzinom, alleine oder in Kombination mit anderen Wirkstoffen/Medikamenten.

## Claims

1. Compounds of the general formula 2, 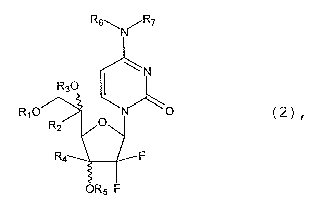 wherein R₁, R₃ and R₅ represent hydrogen or a suitable hydroxy protective group, in particular a benzoyl group, each of R₂ and R₄ represents hydrogen or alkyl with 1 to 6 C-atoms, and each of R₆ and R₇ represents hydrogen or a suitable amino protective group, and the wavy line in each case represents both possible configurations of -OR₃ and/or -OR₅ with regard to the parent substance.

2. Compound according to claim 1, **characterised in that** R₁, R₃ and R₅ represent hydrogen or a benzoyl group, R₂ and R₄ represent hydrogen each, and each of R₆ and R₇ represents hydrogen, acetyl, alkylsilanyl or arylalkylsilanyl with 1 to 6 C-atoms in the alkyl moiety.

3. Compound according to claim 1 or 2, **characterised in that** each of R₁ to R₇ represents hydrogen.

4. Method for producing compounds of the general formula 2 according to any one of claims 1 to 3, **characterised in that** a compound of the general formula 3 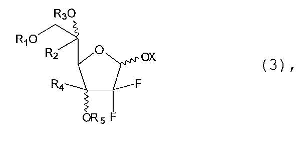 wherein R₁ to R₅ are as defined in claim 1, X represents hydrogen or an activating group known per se, and the wavy line in each case represents both possible configurations of OX, -OR₃ and/or -OR₅ with regard to the parent substance,
is reacted with a cytosine, provided with protective groups, of the general formula 4 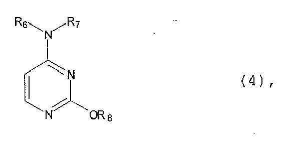 wherein R₆ and R₇ are as defined in claim 1, and R₈ is a suitable leaving group,
wherein, subsequently, any protective groups possibly still present are optionally cleaved to obtain compounds of the general formula (2), wherein each of R₁ to R₇ represents hydrogen.

5. Method according to claim 4, **characterised in that** X represents an alkylsulphonyl residue with 1 to 6 C-atoms in the alkyl moiety, and at least one of R₆ and R₇ represents trialkylsilanyl or triarylalkylsilanyl with 1 to 6 C-atoms each in the alkyl moiety.

6. Method according to claim 4 or 5, **characterised in that** R₈ is equal to R₆ and R₇.

7. Compounds of the general formula 3 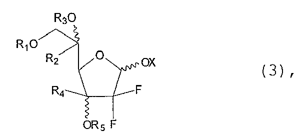 wherein R₁ to R₅ are as defined in claim 1, X represents hydrogen or an activating group known per se, and the wavy line in each case represents both possible configurations of OX, -OR₃ and/or -OR₅ with regard to the parent substance, or the wavy line, together with OX, represents a keto group.

8. Compound according to claim 7, **characterised in that** R₁ represents a suitable hydroxy protective group, R₂ to R₅ represent hydrogen, and the wavy line, together with OX, represents a keto group.

9. Compound according to claim 8, **characterised in that** R₁ represents a benzoyl group.

10. Compound according to claim 7, **characterised in that** R₁, R₃ and R₅ represent a suitable hydroxy protective group, and the wavy line, together with OX, represents a keto group.

11. Compound according to claim 7, **characterised in that** R₁, R₃ and R₅ represent a suitable hydroxy protective group, and X represents hydrogen.

12. Compound according to claim 11, **characterised in that** R₁, R₃ and R₅ represent a benzoyl group.

13. Compound according to claim 7, **characterised in that** R₁, R₃ and R₅ represent a suitable hydroxy protective group, and X represents an activating group.

14. Compound according to claim 13, **characterised in that** R₁, R₃ and R₅ represent a benzoyl group, and X represents an alkylsulphonyl residue with 1 to 6 C-atoms in the alkyl moiety.

15. Method for producing compounds of the general formula 2 according to any one claims 1 to 3, **characterised in that** a compound of the general formula 12 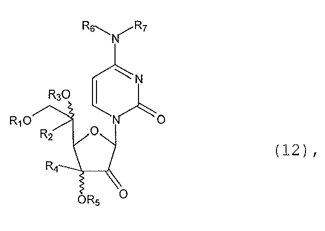 wherein R₁ to R₇ are as defined in claim 1, and the wavy line in each case represents both possible configurations of OX, -OR₃ and/or -OR₅ with regard to the parent substance,
is fluorinated with a suitable fluorinating agent, wherein, subsequently, any protective groups possibly still present are optionally cleaved to obtain compounds of the general formula (2), wherein each of R₁ to R₇ represents hydrogen.

16. Method according to claim 15, **characterised in that** said fluorination is effected with DAST (diethylaminosulphur trichloride) in combination with HF.

17. Method according to any one of claims 15 and 16, **characterised in that** R₁, R₃ and R₅ represent a benzoyl group, and each of R₂, R₄, R₆ and R₇ represents hydrogen.

18. Compound of the general formula 12 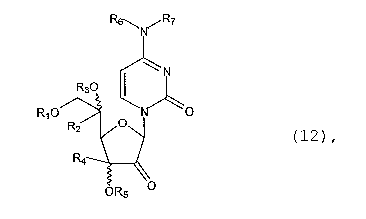 wherein R₁ to R₇ are as defined in claim 1, and the wavy line in each case represents both possible configurations of OX, -OR₃ and/or -OR₅ with regard to the parent substance.

19. Compound according to claim 18, **characterised in that** R₁, R₃ and R₅ represent a benzoyl group, and each of R₂, R₄, R₆ and R₇ represents hydrogen, and at least one of R₆ and R₇ represents hydrogen, trialkylsilanyl or triarylalkylsilanyl with 1 to 6 C-atoms each in the alkyl moiety,

20. Compound according to claim 19, **characterised in that** both R₆ and R₇ represent hydrogen.

21. Method for producing gemcitabine, **characterised in that** a compound according to any one of claims 1 to 3 is optionally deprotected and subjected to a glycol cleavage known per se to the aldehyde of the general formula 7 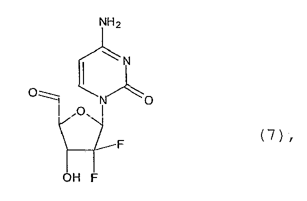 whereupon the aldehyde group of the aldehyde 7 is reduced with a complex hydride, thus obtaining gemcitabine of the formula 1

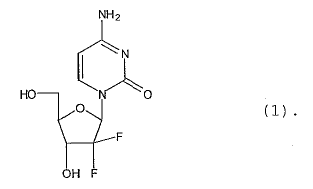

22. Method according claim 21, **characterised in that** the glycol cleavage is effected with a periodate.

23. Method according to any one of claims 21 or 22, **characterised in that** reducing is effected with sodium borohydride.

24. Method according to any one of claims 21 to 23, **characterised in that** in the glycol cleavage the pure β-anomer of compounds of the general formula 2, wherein each of R₁ to R₇ represents hydrogen, is used.

25. Method according to any one of claims 21 ot 23, **characterised in that** in the glycol cleavage a mixture of the α- and β-anomers of compounds of the general formula 2, wherein each of R₁ to R₇ represents hydrogen, is used.

26. Method according to any one of claims 21 o 25, **characterised in that** said reduction is performed in a one-pot reaction after glycol cleavage.

27. Use of a compound of the general formula 2 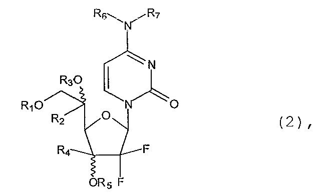 wherein R₁, R₃ and R₅ represent hydrogen, each of R₂ and R₄ represents hydrogen or alkyl with 1 to 6 C-atoms, and each of R₆ and R₇ represents hydrogen or acetyl, and the wavy line in each case represents both possible configurations of -OR₃ and/or -OR₅ with regard to the parent substance, for producing a medicament for treatment of proliferative diseases, in particular for treatment of NSCLC (non-small cell lung cancer), mamma carcinoma, ovarian carcinoma, pancreas carcinoma and bladder carcinoma, individually or in combination with other active substances/medicaments.

28. Use of a compound of the general formula 2 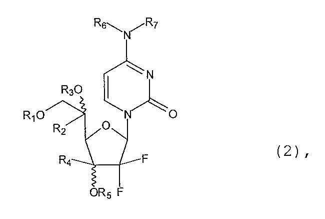 wherein each of R₁ to R₇ represents hydrogen, and the wavy line in each case represents both possible configurations of -OR₃ and/or -OR₅ with regard to the parent substance, for producing a medicament for treatment of proliferative diseases, in particular for treatment of NSCLC (non-small cell lung cancer), mamma carcinoma, ovarian carcinoma, pancreas carcinoma and bladder carcinoma, individually or in combination with other active substances/medicaments.

## Revendications

1. Composés de la formule générale 2 : 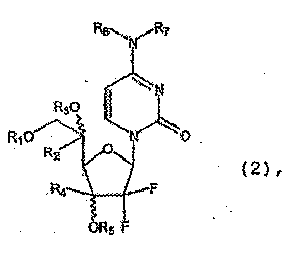 dans laquelle R₁, R₃ et R₅ représentent hydrogène ou un groupe protecteur d'hydroxyle approprié, en particulier un radical benzoyle, R₂ et R₄ représentent chacun, hydrogène ou alkyle ayant 1 à 6 atomes C, et R₆ et R₇ représentent chacun, hydrogène ou un groupe protecteur d'amino approprié, et les traits ondulés signifient chacun, les deux configurations possibles pour -OR₃ et respectivement, -OR₅ par rapport au squelette de base.

2. Composé selon la revendication 1, **caractérisé en ce que** R₁, R₃ et R₅ représentent hydrogène ou un radical benzoyle, R₂ et R₄ représentent chacun, hydrogène, et R₆ et R₇ représentent chacun, hydrogène, acétyle, alkylsilanyle ou arylalkylsilanyle avec 1 à 6 atomes C dans la partie alkyle.

3. Composé selon la revendication 1 ou 2, **caractérisé en ce que** R₁ à R₇ représentent chacun hydrogène.

4. Procédé de préparation de composés de la formule générale 2 selon l'une des revendications 1 à 3, **caractérisé en ce que** l'on fait réagir un composé de la formule générale 3 : 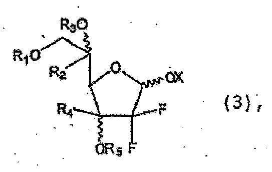 dans laquelle R₁ à R₅ ont la signification donnée à la revendication 1, X représente hydrogène ou un groupe d'activation connu en soi, et les traits ondulés signifient chacun, les deux configurations possibles pour OX, -OR₃ et respectivement, -OR₅ par rapport au squelette de base,
avec une cytosine munie de groupes protecteurs, de la formule générale 4 : 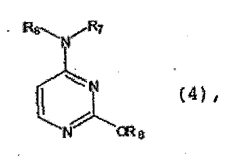 dans laquelle R₆ et R₇ ont les significations données la revendication 1, et R₈ est un groupe partant approprié,
où l'on clive ensuite, tous les groupes protecteurs encore présents, pour obtenir des composés de la formule générale (2), où R₁ à R₇ signifient chacun hydrogène.

5. Procédé selon la revendication 4, **caractérisé en ce que** X signifie un reste alkylsulfonyle ayant 1 à 6 atomes C dans la partie alkyle, et au moins l'un de R₆ et R₇ représente trialkylsilanyle ou triarylalkylsilanyle, ayant chaque fois, 1 à 6 atomes C dans les parties alkyle.

6. Procédé selon la revendication 4 ou 5, **caractérisé en ce que** R₈ est identique à R₆ et R₇.

7. Composés de la formule générale 3 : 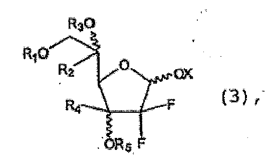 dans laquelle R₁ à R₅ ont la signification donnée à la revendication 1, X représente hydrogène ou un groupe d'activation connu en soi, et les traits ondulés signifient chacun, les deux configurations possibles pour OX, -OR₃ et respectivement, -OR₅ par rapport au squelette de base, ou le trait ondulé de OX représente un radical céto.

8. Composé selon la revendication 7, **caractérisé en ce que** R₁ représente un groupe protecteur d'hydroxyle approprié, R₂ à R₅ représentent hydrogène et le trait ondulé ensemble avec OX représente un radical céto.

9. Composé selon la revendication 7, **caractérisé en ce que** R₁ représente un radical benzoyle.

10. Composé selon la revendication 7, **caractérisé en ce que** R₁, R₃ et R₅ représentent un groupe protecteur d'hydroxyle approprié, et le trait ondulé ensemble avec OX représente un radical céto.

11. Composé selon la revendication 7, **caractérisé en ce que** R₁, R₃ et R₅ représentent un groupe protecteur d'hydroxyle approprié et X représente hydrogène.

12. Composé selon la revendication 11, **caractérisé en ce que** R₁, R₃ et R₅ représentent un radical benzoyle.

13. Composé selon la revendication 7, **caractérisé en ce que** R₁, R₃ et R₅ représentent un groupe protecteur d'hydroxyle approprié, et X représente un groupe d'activation.

14. Composé selon la revendication 13, **caractérisé en ce que** R₁, R₃ et R₅ représentent un radical benzoyle, et X représente un reste alkylsulfonyle ayant 1 à 6 atomes C dans la partie alkyle.

15. Procédé de préparation des composés de la formule générale 2 selon l'une des revendications 1 à 3, **caractérisé en ce que** l'on effectue une fluoration d'un composé de la formule générale 12 : 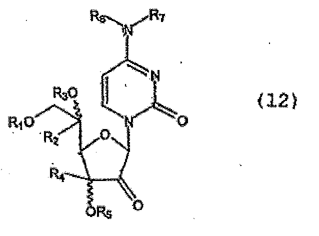 dans laquelle R₁ à R₇ ont la signification donnée à la revendication 1, et les traits ondulés signifient chacun, les deux configurations possibles pour OX, -OR₃ et respectivement, -OR₅ par rapport au squelette de base,
avec un agent de fluoration approprié, où l'on clive ensuite, les groupes protecteurs encore présents, pour obtenir des composés de la formule générale (2) où R₁ à R₇ représentent chacun hydrogène.

16. Procédé selon la revendication 15, **caractérisé en ce que** la fluoration est réalisée avec le DAST (trichlorure de diéthylaminosoufre) en combinaison avec le HF.

17. Procédé selon l'une des revendications 15 et 16, **caractérisé en ce que** R₁, R₃ et R₅ représentent un radical benzoyle et R₂, R₄, R₆ et R₇ représentent chacun hydrogène.

18. Composé de la formule générale 12 : 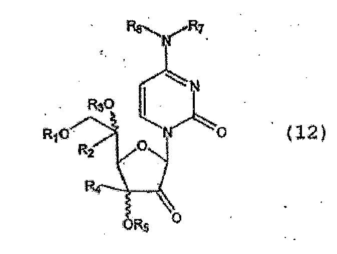 dans laquelle R₁ à R₇ ont la signification donnée à la revendication 1, et les traits ondulés signifient chacun, les deux configurations possibles pour OX, -OR₃ et respectivement, -OR₅ par rapport au squelette de base.

19. Composé selon la revendication 18, **caractérisé en ce que** R₁, R₃ et R₅ représentent un radical benzoyle, R₂, R₄, R₆ et R₇ représentent chacun hydrogène et au moins l'un de R₆ et R₇ représente hydrogène, trialkylsilanyle ou triarylalkylsilanyle ayant 1 à 6 atomes C dans les parties alkyle.

20. Composé selon la revendication 19, **caractérisé en ce que** R₆ et R₇ représentent tous deux hydrogène.

21. Procédé de préparation de gemcitabine, **caractérisé en ce qu'**un composé selon l'une des revendications 1 à 3 est le cas échéant déprotégé et est soumis à un clivage de glycol connu en soi pour donner l'aldéhyde de la formule générale 7 : 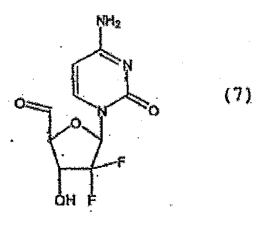 Après quoi le radical aldéhyde de l'aldéhyde 7 est réduit avec un hydrure complexe, ce par quoi on obtient la gemcitabine de la formule 1 :

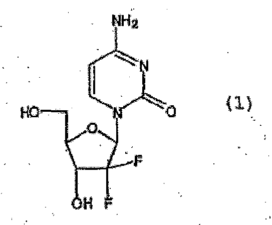

22. Procédé selon la revendication 21, **caractérisé en ce que** le clivage de glycol est réalisé avec un periodate.

23. Procédé selon l'une des revendications 21 ou 22, **caractérisé en ce que** la réduction est réalisée avec le borohydrure de sodium.

24. Procédé selon l'une des revendications 21 à 23, **caractérisé en ce que** dans le clivage de glycol, on met en oeuvre l'anomère □ pur des composés de la formule générale 2, où R₁ à R₇ représentent chacun hydrogène.

25. Procédé selon l'une des revendications 21 à 23, **caractérisé en ce que** dans le clivage de glycol, on met en oeuvre un mélange des anomères □ et □ des composés de la formule générale 2, où R₁ à R₇ représentent chacun hydrogène.

26. Procédé selon l'une des revendications 21 à 25, **caractérisé en ce que** la réduction est réalisée selon un procédé « one pot » à la suite du clivage du glycol.

27. Utilisation d'un composé de la formule générale 2 : 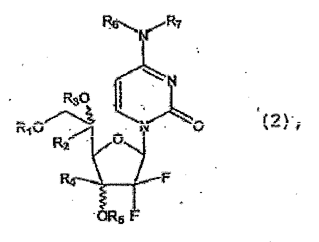 dans laquelle R₁, R₃ et R₅ représentent hydrogène, R₂ et R₄ représentent chacun hydrogène ou alkyle ayant 1 à 6 atomes C, et R₆ et R₇ représentent chacun hydrogène ou acétyle, et les traits ondulés représentent les deux configurations possibles pour -OR₃ et respectivement, -OR₅ par rapport au squelette de base, pour la préparation d'un médicament pour le traitement de maladies prolifératives, en particulier pour le traitement du NSCLC (non-small cell lung cancer, cancer du poumon non à petites cellules), d'un carcinome mammaire, d'un carcinome ovarien, d'un carcinome du pancréas et d'un carcinome de la vessie, seul ou en combinaison avec d'autres agents actifs/médicaments.

28. Utilisation d'un composé de la formule générale 2 : 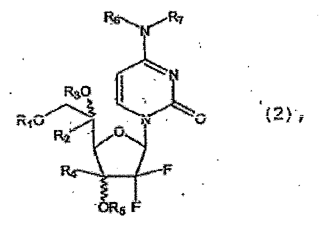 dans laquelle R₁ à R₇ représentent chacun hydrogène, et les traits ondulés représentent les deux configurations possibles pour -OR₃ et respectivement, -OR₅ par rapport au squelette de base, pour la préparation d'un médicament pour le traitement de maladies prolifératives, en particulier pour le traitement du NSCLC (non-small cell lung cancer, cancer du poumon non à petites cellules), d'un carcinome mammaire, d'un carcinome ovarien, d'un carcinome du pancréas et d'un carcinome de la vessie, seul ou en combinaison avec d'autres agents actifs/médicaments.
